# EUROPEAN PATENT SPECIFICATION

(11) **EP 3 439 707 B1**
(45) Date of publication and mention of the grant of the patent: **08.06.2022**
(21) Application number: 17718281.3
(22) Date of filing: 05.04.2017
(51) Int. Cl.: A61L 27/22, A61L 27/38, A61L 27/50, A61L 27/52, A61L 27/60, A61L 31/00, A61L 31/04, A61L 31/14

(54) **ENGINEERED TISSUES HAVING STRUCTURAL COMPONENTS EMBEDDED THEREIN, AND METHODS OF MAKING AND USING**
MANIPULIERTE GEWEBE MIT DARIN EINGEBETTETEN STRUKTURKOMPONENTEN SOWIE VERFAHREN ZUR HERSTELLUNG UND VERWENDUNG
TISSUS MODIFIÉS DANS LESQUELS DES COMPOSANTS STRUCTURAUX SONT INCORPORÉS, ET PROCÉDÉS DE PRÉPARATION ET D'UTILISATION ASSOCIÉS

(30) Priority: 05.04.2016 US 201662318389 P
(43) Date of publication of application: 13.02.2019
(73) Proprietor: Regents of the University of Minnesota, Minneapolis, MN 55455-2020 (US)
(72) Inventor: TRANQUILLO, Robert, Arden Hills, MN 55112 (US); SYEDAIN, Zeeshan, Minneapolis, MN 55414-4450 (US)
(74) Representative: Grund, Martin
(86) International application number: PCT/US2017/026204
(87) International publication number: WO 2017/176919

(56) References cited:
- WO-A1-02/090528
- WO-A1-2010/028197
- WO-A1-2012/111000
- WO-A2-2004/038004
- US-A1- 2014 180 399

## Description

### TECHNICAL FIELD

This disclosure generally relates to engineered tissues having structural components embedded therein, and methods of making and using such engineered tissues having structural components embedded therein.

### BACKGROUND

Suturing tissue to a stent is required for essentially all transcatheter heart valve replacements, yet suturing tissue to a stent is an incredibly tedious, manual, and time-consuming process. For example, suturing tissue to a stent can require up to one week for one professional sewer to create one valve from the industry-standard processed animal tissue, which typically is bovine pericardium. Similar processes also are required to produce replacement venous valves as well as certain vascular grafts.

In addition to significantly reducing or eliminating the time-consuming process of suturing tissue to a stent, the present disclosure provides for structural reinforcement of the engineered tissue.

US 2014/180399 describes a heart valve comprising heart valve leaflets. Said leaflets comprise a mesh material, preferably from nitinol. A bioactive material is used to coat the valve. This bioactive material may be a fibrin gel in combination with cells and growth factors. The construct is cultured to grow a cell layer on it.

### SUMMARY

The present invention is defined by the independent claims, while the dependent claims concern the preferred embodiments. Any "embodiment" or "example" which is disclosed in the description but is not covered by the claims should be considered as presented for illustrative purpose only. This disclosure provides engineered decellularized tissues having structural components embedded therein. This disclosure also provides methods of making and methods of using engineered tissues having structural components embedded therein.

In one aspect, a method of making an engineered decellularized tissue that includes a structural component embedded therein is provided, wherein a portion of the structural component is embedded in the engineered decellularized tissue, and a portion of the structural component is not embedded in said engineered decellularized tissue.

Such a method generally includes combining fibrinogen, thrombin, and matrix-producing cells (e.g., fibroblasts, smooth muscle cells and/or interstitial cells) to produce a cell-seeded hydrogel; forming the cell-seeded hydrogel on, in, around, and/or within a structural component. In some embodiments, the method further includes manipulating, mechanically, the tube in the presence of culture medium to produce an engineered tissue. According to the claimed invention, the method further includes decellularizing the biologically-engineered tubular graft. In some embodiments, the method further includes recellularizing or chemically modifying the engineered tissue.

In another aspect, an engineered decellularized tissue is provided that includes a portion of a structural component embedded therein, wherein a portion of the structural component is not embedded in the engineered decellularized tissue. According to the claimed invention, the structural component is thus partially embedded in the engineered tissue. Representative structural components include, without limitation, a stent, a tab, mesh, or a sheath. In some embodiments, the engineered decellularized tissue is a vascular graft, a heart valve, a vein valve, an arterio-venous graft, an ureter, an urethra, an esophagus a trachea, skin, tendons and ligaments, uterine (e.g., fallopian) tubes, or hernia mesh.

In still another aspect, a composition is provided that includes a structural component embedded within a decellularized engineered tissue. According to the claimed invention, the structural component of such a composition typically includes a first end and a second end, where the first end includes a valve stent and the second end includes a tubular structure. Generally, the tubular structure of the structural component is embedded in the engineered decellularized tissue, whereas the valve stent is not embedded within said engineered decellularized tissue.

In some embodiments, the tubular structure of the structural component includes a valve leaflet wire. In some embodiments, the composition includes a sewing cuff attached to the second end of the structural component. In some embodiments, the structural component is made from nitinol.

Unless otherwise defined, all technical and scientific terms used herein have the same meaning as commonly understood by one of ordinary skill in the art to which the methods and compositions of matter belong. Although methods and materials similar or equivalent to those described herein can be used in the practice or testing of the methods and compositions of matter, suitable methods and materials are described below. In addition, the materials, methods, and examples are illustrative only and not intended to be limiting.

### DESCRIPTION OF DRAWINGS

Figure 1 is a photograph showing one embodiment of an engineered tissue with an embedded wire mesh structure.
Figure 2 is a photograph showing one embodiment of an engineered tissue with two embedded structural components. The arrow on the left shows an embodiment of an engineered tissue with an embedded stent, and the arrow on the right shows an embodiment of an engineered tissue with an embedded wire mesh structure.
Figure 3 is a photograph showing one embodiment of an engineered tissue with an embedded tab.
Figure 4A is an image of an engineered tissue stained with trichrome to show the collagen matrix encapsulating the mesh structure. The hole in the center was the portion of the structure that was removed for cryosectioning.
Figure 4B is a photograph of engineered tissue (white color) with embedded mesh structure (dark).
Figure 5A is a schematic of a wire mesh structural component embedded in an engineered tissue shaped in the form of a tube.
Figure 5B is a schematic of a wire mesh structural component embedded in an engineered tissue shaped in the form of a tube, where one end of the engineered tissue has been anchored to form an engineered valve.
Figure 6A shows an image of a nitinol wire and nitinol stent embedded into engineered tissue.
Figure 6B is an image that shows that, after folding the engineered tissue structure with nitinol wire into the stent, a tri-leaflet aortic valve with an inner diameter of 24 mm is formed. The image of the valve after sewing the folded structure is shown on the right.
Figure 6C is an image of an end on view of the valve from Figure 6B being crimped to a diameter of 8 mm (from an initial diameter of 24 mm).
Figure 6D are images showing tissue material before (left) and after (right) crimping, showing no qualitative tear, cut or peeling of tissue from the embedded structure.
Figure 7A is an image showing a side view of a mold having a tubular cavity for placing the nitinol stent and channels cut into the mold for leaflets.
Figure 7B is an image of a side view of a stent embedded into engineered tissue.
Figure 7C is an image of a top-down view showing two leaflets within the embedded stent.
Figure 7D is an image of an end-on view of leaflets in closed (left) and open (right) positions within the embedded stent.
Figure 8A is a graph showing a back-pressure failure test of the bi-leaflet tissue-engineered vein valve (shown in Figure 7) compared to a native ovine vein valve.
Figure 8B is a graph showing the results of a dynamic flow test of the bi-leaflet tissue-engineered vein valve (shown in Figure 7) compared to a native ovine vein valve.

### DETAILED DESCRIPTION

Provided herein is an engineered decellularized tissue that has one or more structural components embedded within the engineered tissue. The process is based on forming a hydrogel containing cells onto, into, around and/or within one or more structural components. Under suitable culture conditions and in the presence of, for example, thrombin, the cells remodel the hydrogel into tissue, which forms on, in around and/or within the structural component(s), thereby embedding the structural component(s) in the engineered tissue. The manner of embedding the structural component within the engineered tissue and the manner in which the engineered tissue is obtained (e.g., by remodeling of a cell-seeded hydrogel) results in a number of unique features. According to the claimed invention, the structural component is partially lodged or embedded in or within the engineered tissue. If desired, the result can be a structural component or portion thereof that is integrated or entwined in the engineered tissue or a portion thereof.

### Engineered Tissues

The use of hydrogels in tissue engineering is known. See, for example, U.S. Patent No. 6,666,886 and U.S. Publication No. 2014/0058496). Typically, fibrinogen (or a suitable alternative such as a modified fibrinogen), thrombin, and cells capable of producing tissue matrices (e.g., fibroblasts, smooth muscle cells, interstitial cells) are combined in a hydrogel solution, and the resulting cell-seeded hydrogel is molded (or cast, shaped, deposited, formed, or printed) into the desired shape and to appropriately accommodate and embed the structural component therein. The shape of the structural component will determine the extent to which the cell-seeded hydrogel can be molded (or cast, shaped, deposited, formed, or printed) on, in, around and/or within the structural component. Under appropriate culture conditions, the cell-seeded hydrogel is remodeled into an extracellular matrix by the cells, which maintains the original shape and results in the one or more structural components being embedding into the engineered tissue. Based on the manner in which the engineered tissue is produced, the resulting engineered tissue, when examined in a cross-section, is uniform and lacks any layers.

Hydrogel solutions generally refer to aqueous solutions containing hydrophilic polymeric chains that can include natural or synthetic polymers. Representative hydrogels include, for example, agarose, methylcellulose, hyaluronan, and combinations thereof. The amount of hydrogel used in a composition described herein can range from about 1% to about 80% (e.g., about 2% to about 75%, about 5% to about 75%, 20% to about 70%, about 30% to about 60%, about 40% to about 50%, or about 50%). The presence of and the actual amount of one or more hydrogels will depend upon the desired features of the cell-seeded hydrogel or the subsequently produced biologically-engineered tubular graft (e.g., softness or hardness, flexibility, absorbency).

The cells used herein (i.e., in the cell-seeded hydrogel) are those cells that are able to convert fibrinogen, in the presence of thrombin, into extracellular matrix. Fibroblasts are capable of forming tissue matrices and can be used in a hydrogel as described herein, as can any number of cells that are capable of producing tissue matrices or assist in the production of tissue matrices (e.g., smooth muscle cells, interstitial cells). The cells used to seed a hydrogel can be a single cell type (e.g., fibroblasts or smooth muscle cells or interstitial cells) or the cells used to seed a hydrogel can be a multitude of cell types (e.g., fibroblasts and smooth muscle cells, or fibroblasts and interstitial cells, or smooth muscle cells and interstitial cells). Further, the cells can be from any number of organisms (e.g., human, primate, rodent) and any number of tissues (e.g., dermis, lung, connective tissue, kidney). It would be appreciated that the cells can be added to the hydrogel prior to the gel being appropriately shaped or after the gel has been appropriately shaped on, in, around and/or within the structural component.

The size and shape of an engineered tissue as described herein will depend entirely on its intended use and/or the intended use of the structural component. As shown herein, an engineered tissue can have a tubular shape having a desired length and diameter(s), but an engineered tissue also can be flat or curved as needed. Typically, an engineered tissue as described herein includes a first end and a second end, and, when molded (or cast, shaped, deposited, formed, or printed) in a tubular shape, an exterior and interior (or annular) surface, which defines a longitudinal axis.

During remodeling of the cell-seeded hydrogel into an extracellular matrix and, ultimately, the engineered tissue, the hydrogel and/or the extracellular matrix can be mechanically manipulated (or "conditioned") in any number of fashions, including, without limitation, mechanical manipulation (e.g., static culture, circumferential stretching, longitudinal stretching, inflation / distention), electrical stimulation (e.g., field stimulation, AC or DC current), and/or biological manipulation with molecules / compounds such as growth factors and cytokines. Usually, manipulation of the gel / matrix is cyclic or periodic.

According to the claimed invention, once remodeling is complete or near complete, the engineered tissue is decellularized (e.g., to remove the matrix-forming cells). Decellularization is known in the art and can be performed using a number of different methods. See, for example, WO 2007/025233, WO 2010/120539, Ott et al. (2008, Nat. Med., 14:213-21), Baptista et al. (2009, Conf. Proc. IEEE Eng. Med. Biol. Soc., 2009:6526-9) or Crapo et al. (2011, Biomaterials, 32:3233-43). The engineered decellularized tissue can be recellularized with one or more desired cell types. The type(s) of cells used for recellularization will depend on its intended use. Cells used in the recellularization process can be stem cells (e.g., embryonic stem cells (ESCs), induced pluripotent stem cells (iPSCs), mesenchymal stem cells (MSCs), or adult-derived stem cells), or cells used in the recellularization process can be non-stem cells (e.g., one or more types of cells at some stage of differentiation). Simply by way of example, non-stem cells include, without limitation, endothelial cells, epithelial cells, fibroblasts, smooth muscle cells, and/or any tissue- or organ-specific cells (e.g., liver, heart, lung, bone, kidney, pancreas). In some instances, the engineered decellularized tissues can be implanted and recellularized naturally *in vivo.*

Alternatively or additionally, the engineered tissue (i.e. decellularized engineered tissue) can be chemically or physically modified. Chemical modifications to engineered tissue include, for example, matrix crosslinking by chemical agents (e.g., glutaraldehyde) or enzymes (e.g., transglutaminase), or conjugation of proteins (e.g., heparin) or peptides by enzymes or via high affinity domains of fusion proteins. Physical modifications (e.g. for matrix crosslinking) include, without limitation, dehydration, heating, and/or UV irradiation.

Engineered tissues made using hydrogels and the appropriate cells possess a number of beneficial features, such as, without limitation, circumferential alignment of fibers (for tubular structures, which results in a greater tensile stiffness in the circumferential direction than in the longitudinal direction) and a high burst pressure (e.g., at least 3500 mm Hg, at least 4000 mm Hg, or greater than 4000 mm Hg, without bursting).

### Structural Components

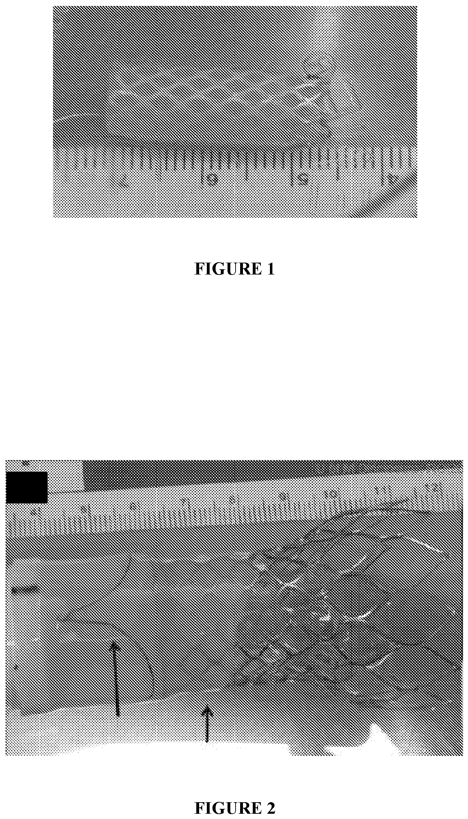

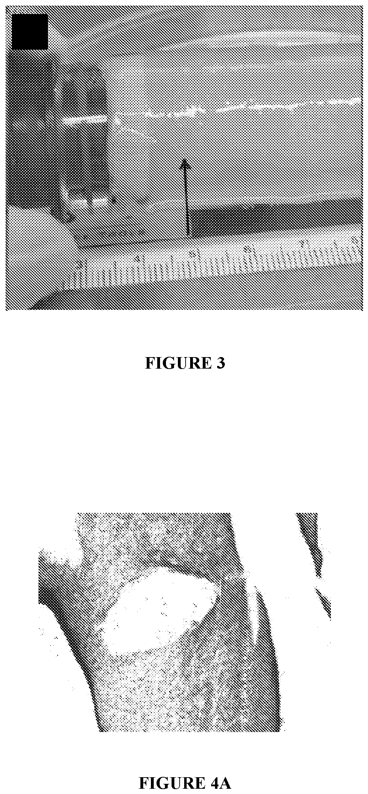

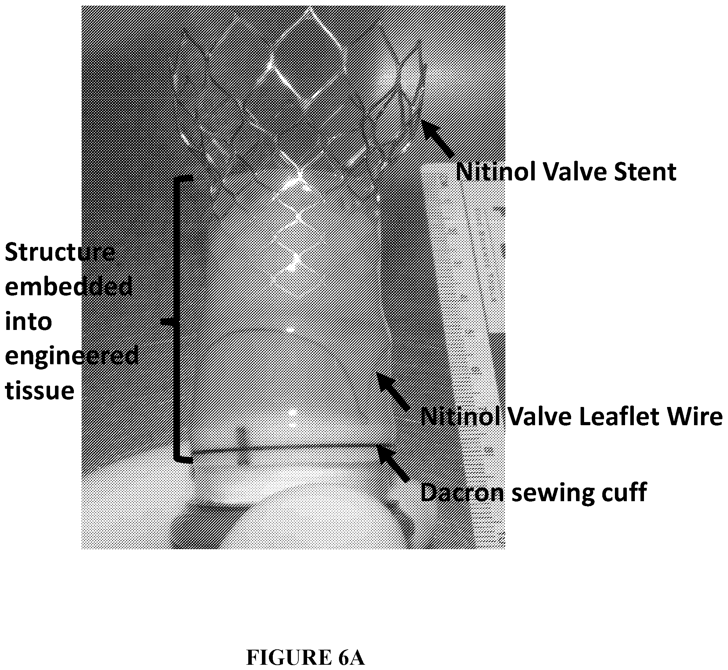

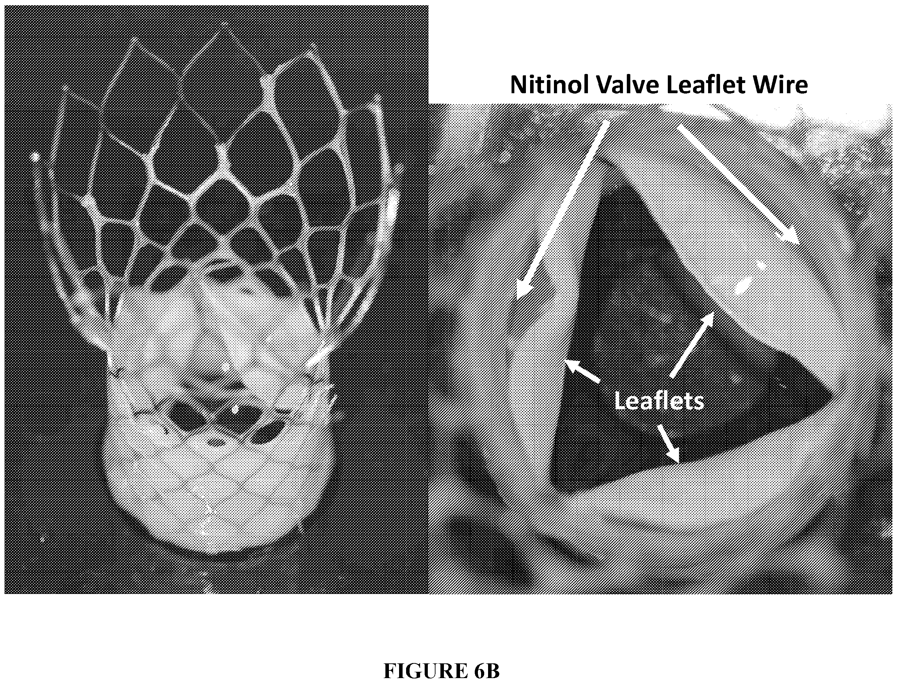

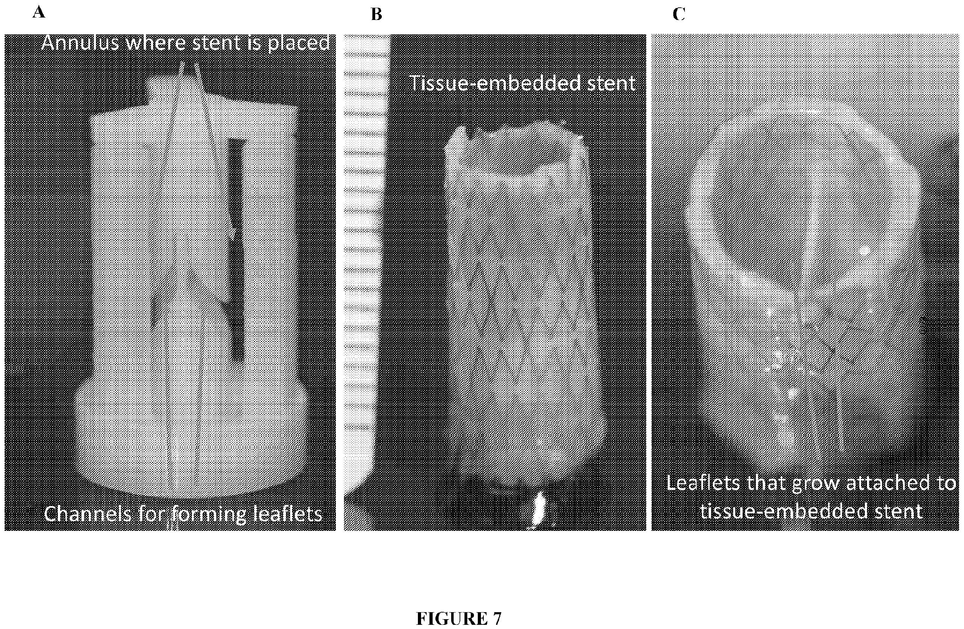

A structural component as described herein can take essentially any shape desired. As shown herein in various embodiments, structural components can be a mesh structure (see Figure 1), a stent (see the right portion of Figure 2, Figure 6A, Figure 7B, and Figure 7C), a tab (see Figure 3), or a leaflet wire (see the left portion of Figure 2, Figure 6A, and Figure 6B). Although not part of the claimed invention, the addition of a structural component that is wholly embedded within the engineered tissue (see, for example, Figure 7C) can enable easy suturing to another object (e.g. a stent), or to a sheath, and increase the strength and integrity of the overall resulting product.

Structural components that are suitable for use herein can be made from materials that are known in the art. Essentially any material can be used that does not cause an immune response in the subject. For example, any of the materials that are used to make stents can be used in a structural component as described herein. Such materials include, without limitation, metals or alloys (e.g., shape memory alloys such as, e.g., nitinol), plastics, ceramics, fabrics, and combinations thereof.

A structural component as described herein can include a first end and a second end, and, when the structural component includes a tubular shape, an exterior and interior, or annular, region, which defines a longitudinal axis. In some instances, the first end of the structural component can be embedded within the first end of an engineered tissue; in some instances, a first structural component can be embedded within the first end of an engineered tissue and a second structural component can be embedded within the second end of the engineered tissue. In some instances not according to the claimed invention, the entirety of a structural component can be embedded within the engineered tissue (see, for example, Figures 1, 7B and 7C), while in some instances which are according to the claimed invention, only a portion of the structural component is embedded within the engineered tissue (see, for example, Figures 2 and 6A).

As described herein, an engineered tissue can contain one or more structural components embedded therein. See, for example, Figure 2, which shows a stent embedded on the left end and a mesh structure embedded on the right end. Also as described herein, the structural component(s) can provide one or more than one functionality. For example, Figure 2 shows an engineered tissue in which the embedded structural components function as a stent and as a means to attach the engineered tissue to existing tissue. It is noted that, while the stent and the wire mesh shown in Figure 2 are separate structural components, both functionalities could be engineered into a single structural component.

### Methods of Using

A decellularized engineered tissue having a structural component partially embedded therein can be implanted into a subject.

Similarly, a decellularized engineered tissue having a structural component partially embedded therein can be recellularized, and the resulting recellularized engineered tissue having a structural component partially embedded therein can be implanted into a subject.

When the shape of the engineered tissue having a structural component embedded therein includes a tubular shape, the engineered tissues having a structural component embedded therein as described herein can be used to replace any part of the vasculature (e.g., a tissue-engineered vascular graft, a tissue-engineered heart valve, a tissue-engineered vein valve, a tissue-engineered arterio-venous graft) or other tubular structures (e.g., a tissue-engineered ureter, a tissue-engineered urethra, a tissue-engineered uterine (e.g., fallopian) tube), a tissue-engineered esophagus, a tissue-engineered trachea). Neither the engineered tissue nor the structural component that is embedded therein, however, is limited to a tubular shape, and, thus, can be used for virtually any application that involves a biological tissue (e.g., tissue-engineered tendons and ligaments, tissue-engineered skin, tissue-engineered hernia mesh (for repair)).

In some instances, when the shape of the engineered tissue having a structural component embedded therein includes a tubular shape, an engineered valve can be created by anchoring the tissue at one end of the engineered tissue portion at two points (for a bi-leaflet valve), three points (for a tri-leaflet valve) or four points (for a quad-leaflet valve). Anchoring can be achieved using, for example, one or more sutures through or around the embedded component to attach the tissue to a surrounding stent. See, for example, Figure 6B.

In accordance with the present invention, there may be employed conventional molecular biology, microbiology, biochemical, and recombinant DNA techniques within the skill of the art. Such techniques are explained fully in the literature. The invention will be further described in the following examples. These examples are to be considered as reference-examples which are included for illustrative purposes only, as they do not disclose an engineered decellularized tissue comprising a structural component which is only partially embedded therein.

### REFERENCE-EXAMPLES

### Example 1-Engineered Tissue Having a Structural Mesh Embedded Therein

Figure 1 shows one embodiment of an engineered tissue having an embedded mesh component. The embodiment shown in Figure 1 used an aluminum mesh (McMaster Carr), which was rolled into a tube and the two long ends were tied using silk sutures (Fine Science Tools) to form a tubular structure having a diameter of 6 mm. The aluminum tubular structure was then inserted around a glass rod (McMaster Carr) having a diameter of 4 mm, leaving a cavity of 1 mm around the circumference. This was then inserted into a glass shell having an inner diameter of 8 mm (McMaster Carr). A fibrinogen-based mixture comprising 4 mg/ml fibrinogen (Sigma), 1 million cells neonatal dermal fibroblasts (Lonza), and 0.8 units/ml of thrombin (Sigma) was injected into the cavity formed by the glass rod and shell and having the aluminum mesh suspended in between. Once the fibrinogen formed a fibrin gel due to the enzymatic action of thrombin, the outer glass shell and inner glass tube was removed, leaving behind an aluminum tubular mesh embedded within a formed fibrin gel.

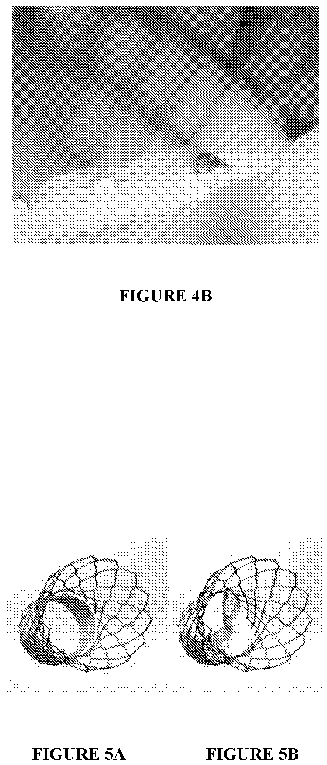

The structure was then cultured in a polycarbonate jar (Nelgene) filled with 50 ml of DMEM media (Gibco) with 10% Fetal Bovine Serum (HyClone) supplemented with insulin (Sigma) and ascorbic acid (Sigma). The image shows the resulting structure on day 3. The structure was cultured for additional 18 days for a total of 21 days of culture in media. The sample was then cut open with scissors and the image was taken to demonstrate that the aluminum structure was embedded within the engineered tissue (Figure 4B). The aluminum was removed and tissue was fixed in 4% paraformaldehyde. The fixed tissue was cryosectioned and stained with trichrome stain (leaving behind the hole visible in Figure 4A). The fibroblast cell nuclei are stained black and the cell-produced collagen is stained green. The original fibrin, which would have stained red, is not visible, indicating that the original fibrin gel was completely degraded by the cells as they formed the collagenous tissue.

### Example 2-Engineered Tissue Having a Nitinol Wireform and Nitinol Stent Embedded Therein

Figure 2 shows one embodiment of an engineered tissue with an embedded Nitinol wireform and Nitinol stent. The embodiment shown in Figure 2 used a Nitinol wireform having a diameter of 0.254 mm (0.01 inch), which was formed into a crown shape with three peaks and three valleys having an inner diameter of 24 mm, and then heat set at 54°C for 40 minutes to shape set the nitinol. In addition, a Nitinol stent was formed by expanding a Nitinol tube and laser cutting holes to form a mesh with diamond shaped holes and having an inner diameter of 24 mm. The two Nitinol structures were sleeved over a glass rod (McMaster Carr) having a diameter of 22 mm. The glass rod and Nitinol structures were placed into a polycarbonate tube having an inner diameter of 28 mm (McMaster Car). A cell suspension comprising 4 mg/ml fibrinogen (Sigma), 1 million cell dermal fibroblast (Coreal), and 0.8 Units/ml thrombin (Sigma) was then injected into the cavity formed by the glass rod and shell and having the Nitinol wireform and stent suspended in between. Once the fibrinogen formed fibrin gel due to the enzymatic action of thrombin, the outer polycarbonate shell was removed, leaving behind the Nitinol structures embedded within a fibrin gel tube formed around the inner glass rod. This was placed in a polycarbonate jar (Nelgene) filled with 250 ml of DMEM media (Gibco) with 10% Fetal Bovine Serum (HyClone) supplemented with insulin (Sigma) and ascorbic acid (Sigma). The image shows the structure on day 7 after manufacturing. The structure was cultured for an additional 28 days for a total of 35 days.

### Example 3-Engineered Tissue Having a Nylon Tab Embedded Therein

Figure 3 shows one embodiment of an engineered tissue with an embedded nylon tab. The embodiment shown in Figure 3 used a laser cut nylon tab having an oval shape and three holes drilled into the central region of the tab. Overall, the tab was 3 mm in length and 1.5 mm in width. Each of the three holes had a diameter of 0.4 mm. Three tabs were suspended into a cavity formed by an inner glass rod (McMaster Carr) having a diameter of 24 mm and an outer polycarbonate shell (McMaster Car) having an inner diameter of 28 mm. A fibrinogen-based mixture comprising 4 mg/ml fibrinogen (Sigma), 1 million cells of dermal fibroblasts (Coreal) and 0.8 Units/ml thrombin (Sigma) was then injected into the cavity formed by the glass rod and shell and having the tabs suspended in between. Once the fibrinogen was formed into a fibrin gel in the presence of the thrombin catalyst, the outer polycarbonate shell was removed, leaving behind the nylon tabs embedded within a formed fibrin gel. The inner glass rod was kept for structures stability and the entire structure was placed in a polycarbonate jar (Nelgene) filled with 250 ml of DMEM media (Gibco) with 10% Fetal Bovine Serum (HyClone) supplemented with insulin (Sigma) and ascorbic acid (Sigma). The image shows the structure on day 7 after manufacturing. The structure was cultured for an additional 28 days for a total of 35 days of culture in media.

### Example 4-Engineered Tissue Having a Valve Stent and Wire Form Embedded Therein

A nitinol valve stent and wire form was embedded in engineered tissue by placing a laser cut nitinol stent and a heat-formed nitinol wire in a tubular mold (Figure 6A). The mold was filled with a hydrogel solution containing fibrinogen, thrombin and dermal fibroblasts.

Once the gel was formed, the embedded nitinol structure was removed from the mold and cultured in cell culture media for 7 weeks.

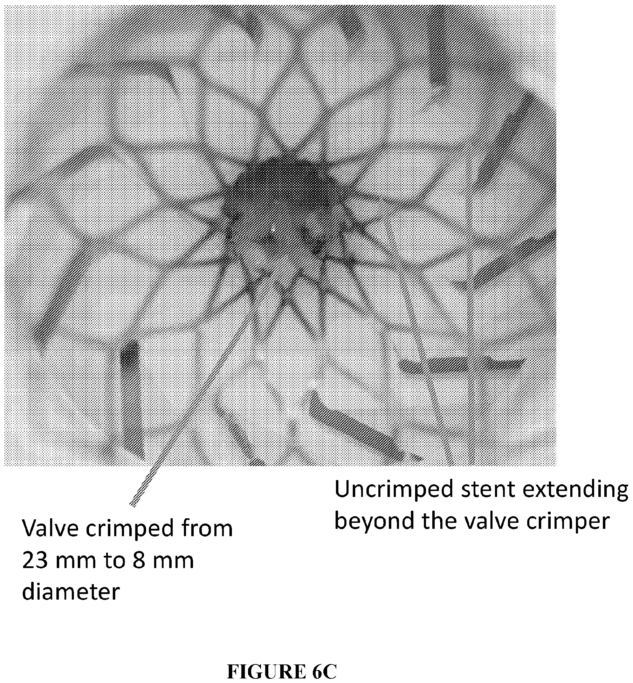

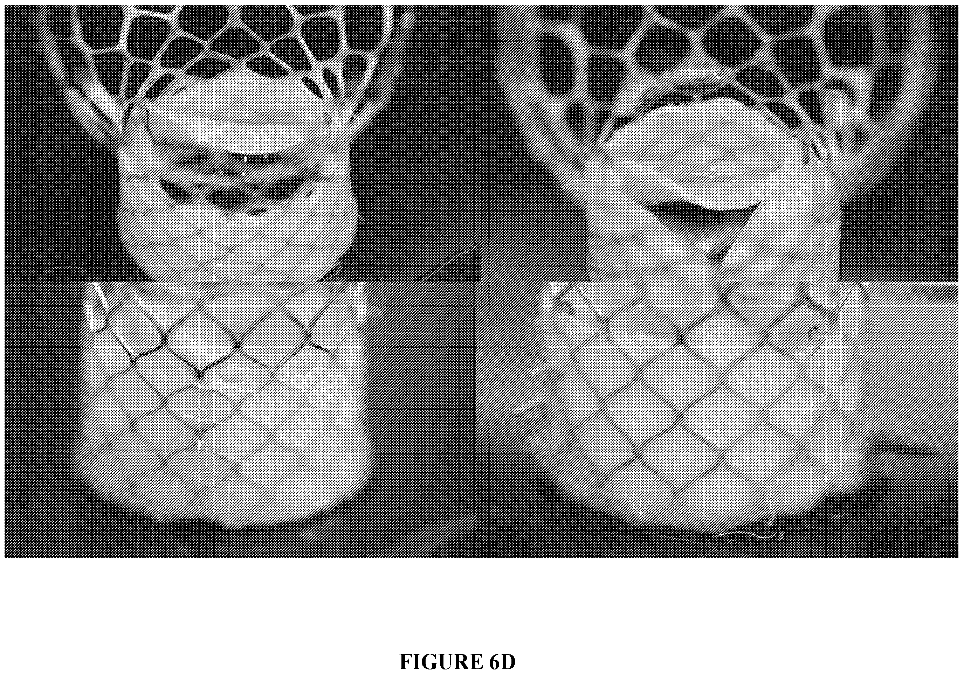

The final product was a cell produced extracellular matrix containing a nitinol structure embedded therein as shown in Figure 6B. The valve was formed by inverting the wire form inside the lumen of the embedded nitinol frame (Figure 6C). To evaluate the integration between the nitinol stent and the surrounding cell-produced extracellular matrix, the stent was crimped from a diameter of about 24 mm to a diameter of about 8 mm (Figure 6D). After being crimped for 15 minutes, the stent was removed and the structure was evaluated for qualitative damage. No tissue tearing, peeling or cuts were seen following crimping of the nitinol stent embedded within the engineered tissue (Figure 6D, right).

### Example 5-Engineered Tissue Having A Vascular Stent Embedded Therein

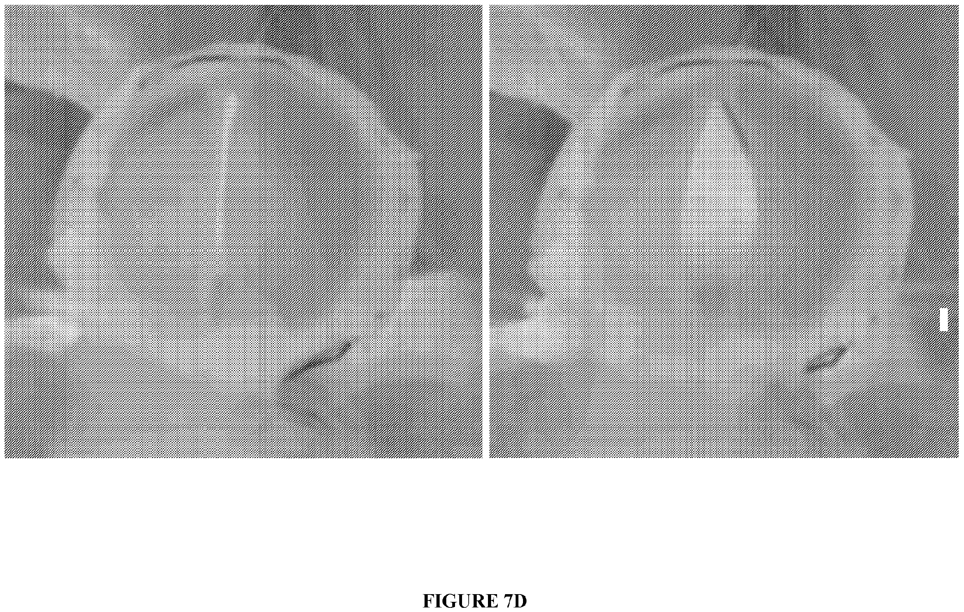

Small diameter nitinol vascular stents (14 mm internal diameter (ID)) were embedded in engineered tissue by placing nitinol stent in a tubular plastic mold with channels for leaflets (Figure 7A). The mold was filled with gelation solution containing fibrinogen, thrombin and dermal fibroblast. Once the gel is formed, the embedded nitinol stent is cultured in cell culture media for 7 weeks. The final product is cell produced extracellular matrix containing a nitinol stent as luminal wall with leaflets (two) in the central lumen as shown in Figure 7B and 7C. The valve end-on images during open and close are shown in Figure 7D.

### Example 6-Valve Performance Testing

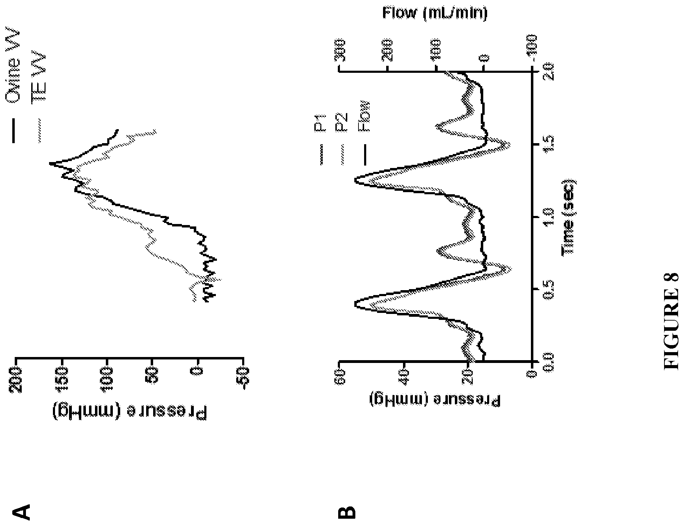

Figure 8 shows the results of performance testing of the bi-leaflet tissue-engineered vein valve shown in Figure 7. The valve was evaluated for hemodynamics performance by mounting in a pulse duplicator (ViVitro System). Figure 8A shows the results from a failure test, in which the back-pressure of the engineered valve is comparable to a native ovine vein valve. Figure 8B shows the results from a dynamic flow test, showing cyclic flow and noflow phases as the pressure gradient reverses, and a systolic pressure gradient of only 1-2 mm Hg.

## Claims

1. A method of making an engineered decellularized tissue comprising a portion of a structural component embedded therein, comprising:
combining matrix-producing cells with a hydrogel to produce a cell-seeded hydrogel, wherein the hydrogel comprises fibrinogen and thrombin;
forming the cell-seeded hydrogel on, in, around, and/or within a portion of a structural component; and
culturing and decellularizing the cell-seeded hydrogel under appropriate conditions so as to produce an engineered decellularized tissue comprising a portion of the structural component embedded therein, wherein a portion of the structural component is not embedded in the engineered decellularized tissue.

2. The method of claim 1, wherein the matrix-producing cells are fibroblasts, smooth muscle cells, or interstitial cells.

3. The method of any of claims 1-2, wherein the structural component is a stent, a tab, mesh, or a sheath,
wherein optionally the structural component is comprised of metal or alloy, plastic, ceramic, fabric or a combination thereof.

4. The method of any of claims 1-3, further comprising manipulating, mechanically, the hydrogel in the culturing step to produce the engineered decellularized tissue.

5. The method of any of claims 1-4, further comprising recellularizing or chemically modifying the engineered decellularized tissue.

6. An engineered decellularized tissue comprising a portion of a structural component embedded therein, wherein a portion of the structural component is not embedded in the engineered decellularized tissue, obtained by the method of any of claims 1-5.

7. The engineered decellularized tissue of claim 6, wherein optionally the structural component is a stent, a tab, mesh, or a sheath,
wherein optionally the structural component is comprised of metal or alloy, plastic, ceramic, fabric or a combination thereof.

8. The engineered decellularized tissue of any of claims 6-7, wherein the engineered decellularized tissue is selected from the group consisting of a vascular graft, a heart valve, a vein valve, an arterio-venous graft, an ureter, an urethra, an esophagus, a trachea, skin, tendons and ligaments, uterine (e.g., fallopian) tube, and hernia mesh.

9. The engineered decellularized tissue of claim 6,
wherein the structural component comprises a first end and a second end, wherein the first end comprises a valve stent and the second end comprises a tubular structure;
wherein the tubular structure of the structural component is embedded in the engineered decellularized tissue and
wherein the valve stent is not embedded in the engineered decellularized tissue.

10. The engineered decellularized tissue of claim 9, wherein the tubular structure of the structural component comprises a valve leaflet wire.

11. The engineered decellularized tissue of claim 9 or 10, comprising a sewing cuff attached to the second end of the structural component.

12. The engineered decellularized tissue of claims 9-11, wherein the structural component is comprised of nitinol.

## Patentansprüche

1. Ein Verfahren zur Herstellung eines manipulierten dezellularisierten Gewebes umfassend einen darin eingebetteten Teil einer strukturellen Komponente, umfassend:
Kombinieren Matrix-produzierender Zellen mit einem Hydrogel, um ein zellbesätes Hydrogel zu bilden, wobei das Hydrogel Fibrinogen und Thrombin umfasst;
Formen des zellbesäten Hydrogels auf, in, um und/oder innerhalb eines Teils der strukturellen Komponente; und
Kultivieren und Dezellularisieren des zellbesäten Hydrogels unter geeigneten Bedingungen, so dass ein manipuliertes dezellularisiertes Gewebe erzeugt wird, das einen darin eingebetteten Teil der strukturellen Komponente umfasst, wobei ein Teil der strukturellen Komponente nicht im manipulierten dezellularisierten Gewebe eingebettet ist.

2. Das Verfahren aus Anspruch 1, wobei die Matrix-produzierenden Zellen Fibroblasten, Glattmuskelzellen oder interstitielle Zellen sind.

3. Das Verfahren aus irgendeinem der Ansprüche 1-2, wobei die strukturelle Komponente ein Stent, ein Tab, ein Netz oder eine Hülle ist,
wobei die strukturelle Komponente optional aus Metall oder Legierung, Plastik, Keramik, Gewebe oder einer Kombination davon besteht.

4. Das Verfahren aus irgendeinem der Ansprüche 1-3, weiter umfassend mechanisches Manipulieren des Hydrogels im Kultivierungsschritt, um das manipulierte dezellularisierte Gewebe herzustellen.

5. Das Verfahren aus irgendeinem der Ansprüche 1-4, weiter umfassend Rezellularisieren oder chemisches Modifizieren des manipulierten dezellularisierten Gewebes.

6. Ein manipuliertes dezellularisiertes Gewebe umfassend einen darin eingebetteten Teil einer strukturellen Komponente, wobei ein Teil der strukturellen Komponente nicht im manipulierten dezellularisierten Gewebe eingebettet ist, erhalten durch das Verfahren aus irgendeinem der Ansprüche 1-5.

7. Das manipulierte dezellularisierte Gewebe aus Anspruch 6, wobei die strukturelle Komponente optional ein Stent, ein Tab, ein Netz oder eine Hülle ist,
wobei die strukturelle Komponente optional aus Metall oder Legierung, Plastik, Keramik, Gewebe oder einer Kombination davon besteht.

8. Das manipulierte dezellularisierte Gewebe aus irgendeinem der Ansprüche 6-7, wobei das manipulierte dezellularisierte Gewebe ausgewählt ist aus der Gruppe bestehend aus einem Gefäßimplantat, einer Herzklappe, einer Venenklappe, einem arterio-venalen Implantat, einem Harnleiter, einer Harnröhre, einer Speiseröhre, einer Luftröhre, Haut, Sehnen und Bändern, Uterusröhre (z.B. Harnleiter) und Herniennetz.

9. Das manipulierte dezellularisierte Gewebe aus Anspruch 6,
wobei die strukturelle Komponente ein erstes und ein zweites Ende umfasst, wobei das erste Ende einen Klappenstent umfasst und das zweite Ende eine röhrenförmige Struktur umfasst;
wobei die röhrenförmige Struktur der strukturellen Komponente im manipulierten dezellularisierten Gewebe eingebettet ist,
wobei der Klappenstent nicht im manipulierten dezellularisierten Gewebe eingebettet ist.

10. Das manipulierte dezellularisierte Gewebe aus Anspruch 9, wobei die röhrenförmige Struktur der strukturellen Komponente einen Klappenflügeldraht umfasst.

11. Das manipulierte dezellularisierte Gewebe aus Anspruch 9 oder 10, umfassend eine am zweiten Ende der strukturellen Komponente befestigte Nahtmanschette.

12. Das manipulierte dezellularisierte Gewebe aus Ansprüchen 9-11, wobei die strukturelle Komponente aus Nitinol besteht.

## Revendications

1. Procédé de fabrication d'un tissu décellularisé modifié comprenant une partie d'un composant structurel incorporé dans celui-ci, comprenant :
la combinaison de cellules productrices de matrice avec un hydrogel afin de produire un hydrogel ensemencé de cellules, dans lequel l'hydrogel comprend du fibrinogène et de la thrombine ;
la formation de l'hydrogel ensemencé de cellules sur, dans, autour et/ou à l'intérieur d'une partie d'un composant structurel ; et
la culture et la décellularisation de l'hydrogel ensemencé de cellules dans des conditions appropriées de manière à produire un tissu décellularisé modifié comprenant une partie du composant structurel incorporé dans celui-ci, dans lequel une partie du composant structurel n'est pas incorporée dans le tissu décellularisé modifié.

2. Procédé selon la revendication 1, dans lequel les cellules productrices de matrice sont des fibroblastes, des cellules de muscles lisses ou des cellules interstitielles.

3. Procédé selon l'une quelconque des revendications 1 et 2, dans lequel le composant structurel est une endoprothèse, une languette, une maille ou une gaine,
dans lequel, facultativement, le composant structurel est constitué de métal ou d'alliage, de plastique, de céramique, de tissu ou d'une combinaison de ceux-ci.

4. Procédé selon l'une quelconque des revendications 1 à 3, comprenant en outre la manipulation, mécanique, de l'hydrogel dans l'étape de culture afin de produire le tissu décellularisé modifié.

5. Procédé selon l'une quelconque des revendications 1 à 4, comprenant en outre la recellularisation ou la modification chimique du tissu décellularisé modifié.

6. Tissu décellularisé modifié comprenant une partie d'un composant structurel incorporé dans celui-ci, dans lequel une partie du composant structurel n'est pas incorporée dans le tissu décellularisé modifié, obtenu par le procédé selon l'une quelconque des revendications 1 à 5.

7. Tissu décellularisé modifié selon la revendication 6, dans lequel, facultativement, le composant structurel est une endoprothèse, une languette, une maille ou une gaine,
dans lequel, facultativement, le composant structurel est constitué de métal ou d'alliage, de plastique, de céramique, de tissu ou d'une combinaison de ceux-ci.

8. Tissu décellularisé modifié selon l'une quelconque des revendications 6 et 7, dans lequel le tissu décellularisé modifié est choisi dans le groupe constitué d'un greffon vasculaire, d'une valve cardiaque, d'une valve veineuse, d'un greffon artérioveineux, d'un uretère, d'un urètre, d'un œsophage, d'une trachée, de peau, de tendons et de ligaments, d'une trompe utérine (par exemple, de Fallope) et d'une maille pour hernie.

9. Tissu décellularisé modifié selon la revendication 6,
dans lequel le composant structurel comprend une première extrémité et une seconde extrémité, dans lequel la première extrémité comprend une endoprothèse de valve et la seconde extrémité comprend une structure tubulaire ;
dans lequel la structure tubulaire du composant structurel est incorporée dans le tissu décellularisé modifié et
dans lequel l'endoprothèse de valve n'est pas incorporée dans le tissu décellularisé modifié.

10. Tissu décellularisé modifié selon la revendication 9, dans lequel la structure tubulaire du composant structurel comprend un fil de feuillet de valve.

11. Tissu décellularisé modifié selon la revendication 9 ou 10, comprenant un manchon de suture fixé à la seconde extrémité du composant structurel.

12. Tissu décellularisé modifié selon l'une quelconque des revendications 9 à 11, dans lequel le composant structurel est constitué de nitinol.
